(19) Europäisches Patentamt
European Patent Office
Office européen des brevets

(11) Veröffentlichungsnummer: **0 234 586 B1**

(12) **EUROPÄISCHE PATENTSCHRIFT**

(45) Veröffentlichungstag der Patentschrift: **08.11.89**

(51) Int. Cl.⁴: **C07C 67/475**, C07C 69/63, B01J 31/22

(21) Anmeldenummer: **87102770.2**

(22) Anmeldetag: **26.02.87**

(54) **Verfahren zur Herstellung von alpha-Chlor-carbonylverbindungen.**

(30) Priorität: **27.02.86 DE 3606263**

(43) Veröffentlichungstag der Anmeldung: **02.09.87 Patentblatt 87/36**

(45) Bekanntmachung des Hinweises auf die Patenterteilung: **08.11.89 Patentblatt 89/45**

(84) Benannte Vertragsstaaten: **CH DE FR GB LI**

(56) Entgegenhaltungen:
**EP-A- 0 037 474**

**BULLETIN DE LA SOCIETE CHIMIQUE DE FRANCE, Nr. 6, 1970, Seiten 2370-2376, J.-P. DECOR et al.: "Chlorhydrines hétérocycliques. VIII. - Structure et propriétés de chlorhydrines tétrahydrofuranniques"**

(73) Patentinhaber: **BASF Aktiengesellschaft, Carl-Bosch-Strasse 38, D-6700 Ludwigshafen(DE)**

(72) Erfinder: **Eckhardt, Heinz, Dr., Rüdigerstrasse 7, D-6700 Ludwigshafen(DE)**
Erfinder: **Halbritter, Klaus, Dr., Schwarzwaldstrasse 19, D-6800 Mannheim 1(DE)**
Erfinder: **Pape, Frank-Friedrich, Dr., Berner Weg 34, D-6700 Ludwigshafen(DE)**

**EP 0 234 586 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von α-Chlor-carbonylverbindungen. Diese sind als Zwischenprodukte zur Synthese von heterocyclischen Verbindungen brauchbar.

Die nach dem erfindungsgemäßen Verfahren herstellbaren α-Chlor-carbonylverbindungen entsprechen der allgemeinen Formel I:

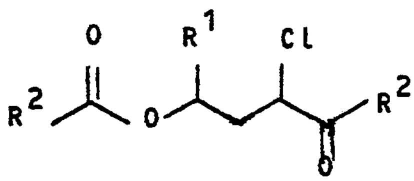

(I)

worin $R^1$ für Wasserstoff oder eine Alkylgruppe mit 1 bis 6 Kohlenstoffatomen steht und die Reste $R^2$ gleich oder verschieden sind und eine Alkylgruppe mit 1 bis 4 Kohlenstoffatomen oder Alkoxygruppe mit 1 bis 4 Kohlenstoffatomen darstellen.

Wenn in der obigen allgemeinen Formel I der Rest $R^1$ für Wasserstoff steht und $R^2$ eine Methylgruppe darstellt, handelt es sich um ein wichtiges Zwischenprodukt für die technische Synthese des Vitamins $B_1$, das (vgl. Ullmanns Enzyclopädie der Technischen Chemie, Band 23, Seite 658, 4. Auflage, 1976) nach der folgenden mehrstufigen Synthese hergestellt wird:

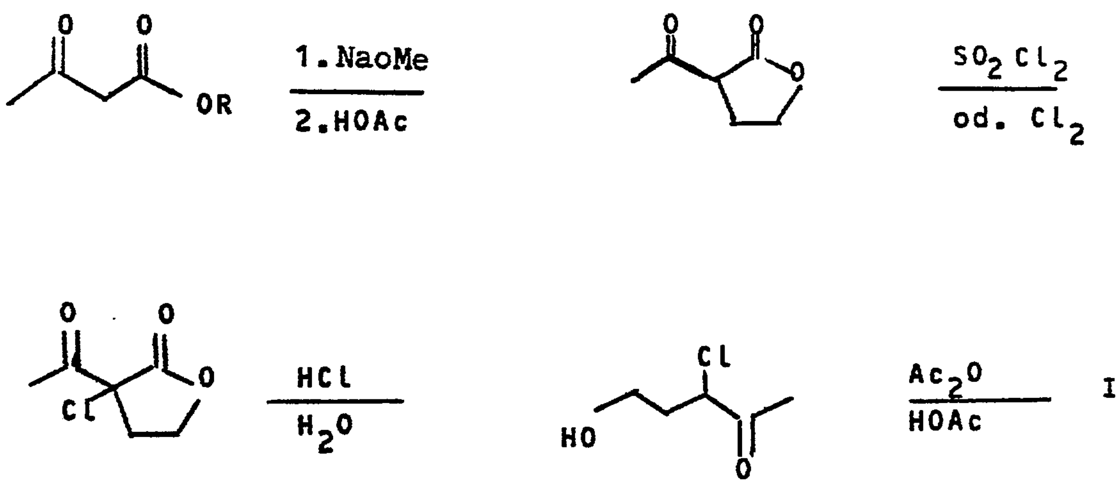


Gerade die erste Stufe dieser Sequenz zeigt große Nachteile infolge der notwendigen Anwendung mindestens stöchiometrischer Mengen an Alkalialkoholat und einem Überschuß an Acetessigester. Da bei niederen Temperaturen gearbeitet werden muß, beeinträchtigen die zunächst salz artig anfallenden, schlecht löslichen Reaktionsprodukte erheblich die Rührbarkeit des Reaktionsgemisches.

Der Erfindung liegt die Aufgabe zugrunde, ein Verfahren der eingangs genannten Art zur Verfügung zu stellen, das im Vergleich zur 4-stufigen Reaktion des Standes der Technik wesentlich vereinfacht ist.

Diese Aufgabe wird gelöst durch ein Verfahren der eingangs genannten Art zur Herstellung von α-Chlor-carbonylverbindungen der allgemeinen Formel I

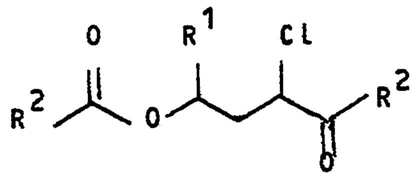

(I)

worin $R^1$ für Wasserstoff oder eine Alkylgruppe mit 1 bis 6 Kohlenstoffatomen steht und die Reste $R^2$ gleich oder verschieden sind und eine Alkylgruppe mit 1 bis 4 Kohlenstoffatomen oder Alkoxygruppe mit 1 bis 4 Kohlenstoffatomen darstellen, das dadurch gekennzeichnet ist, daß man eine α-Chlor-ß-dicarbonylverbindung der allgemeinen Formel II

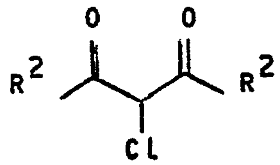

(II)

worin die Reste $R^2$ die vorstehenden Bedeutungen besitzen, mit einem Epoxid der allgemeinen Formel III

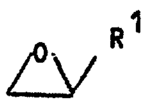

(III)

worin $R^1$ die vorstehenden Bedeutungen besitzt, bei einer Temperatur im Bereich von 2O bis 2OO°C in Gegenwart eines Metallacetylacetonats als Katalysator umsetzt.

Bei einer bevorzugten Ausführungsformel des erfindungsgemäßen Verfahrens setzt man als Katalysatoren aus der Gruppe der Metall-Acetylacetonate solche ein, die als Metalle Al, V, Mn, Mg, Sn, Co, Ni, Fe, Cu, Zr oder Pd, enthalten.

Durch die erfindungsgemäße Umsetzung läßt sich die bekannte vierstufige Reaktion auf nur zwei Stufen verkürzen und somit wesentlich vereinfachen. Durch Chlorierung des leicht verfügbaren Pentandions-2,4 (Acetylaceton), die nach Literaturvorschrift (Neber, Woerner; L. Ann. Chem. Bd. 526, S. 181 (1936) eine sehr gute Ausbeute an der entsprechenden Chlorverbindung liefert, und nachfolgende Umsetzung mit Ethylenoxid in Gegenwart der genannten Katalysatoren erhält man direkt das gewünschte Produkt. Beide Stufen können gegebenenfalls ohne Verwendung eines Lösungsmittels durchgeführt werden, und das Reaktionsgemisch bleibt im gesamten Reaktionsverlauf flüssig und homogen, so daß keine verfahrenstechnischen Schwierigkeiten auftreten.

Die Vorteile der erfindungsgemäßen Umsetzung liegen also im Einsparen zweier Reaktionsstufen, im Verzicht auf Lösungsmittel, bzw. deren Wiedergewinnung und in der Minderung verfahrenstechnischer Probleme. Damit verbunden ist eine Einsparung von Reaktionszeit, an Apparaturen und an Hilfsreagentien (Alkalialkoholat, Essigsäure, Salzsäure, Acetanhydrid).

Die erfindungsgemäße Umsetzung ist neu und war nach dem Stand der Technik auch nicht zu erwarten, da für die Umsetzung von CH-aciden Verbindungen wie Pendandion-2,4 oder Acetessigester mit Ethylenoxid in der Literatur (Houben-Weyl Bd. 6/2, S. 64O ff u. 7OO ff, 4. Auflage) starke Basen wie Alkoholate oder tert. Amine empfohlen werden, die in der Regel auch stöchiometrisch einzusetzen sind. Halogenierte Ausgangsverbindungen können mit diesen Basen aber nicht in der gewünschten Weise umgesetzt werden, da das Halogen dabei verdrängt wird - entweder in Form einer Alkylierung des Amins oder in Art der Williamson-Ethersynthese durch die Reaktion von Alkoholat-Ionen mit der Halogenverbindung. Selbst so schwache Basen wie Metallcarboxylate setzen sich unter Substitution des Halogens um. Ein Beispiel hierfür wird von T. Ishimaru in Chem. Lett. 1977, 1313 gegeben, bei dem 3-Chlorpentandion-2,4 mit Alkalibenzoat bei Raumtemperatur unter Verdrängung des Chloratoms zu 3-Benzoyloxy-pentandion-2,4 in sehr hoher Ausbeute umgesetzt wird. Da die erfindungsgemäß eingesetzten Metallacetylacetonate eine deutlich höhere Basizität besitzen als Metallcarboxylate (Acetylaceton: pKs - 1O; Benzoesäure: pKs - 4,2), war es sehr überraschend, daß hier keine Verdrängung des Halogenatoms, sondern die gewünschte Umsetzung mit dem Epoxid gefunden wurde.

Die Umsetzung kann bei Temperaturen von 2O - 2OO°C durchgeführt werden, bevorzugt werden 5O - 15O°C.

Die geeigneten Reaktionszeiten werden durch die eingestellte Reaktionstemperatur bestimmt; sie betragen zweckmäßig 1 - 1OO Stunden, vorzugsweise 6 - 6O Stunden.

Die Umsetzung wird vorzugsweise bei einem Druck von 1 - 15O bar, zweckmäßig bei dem sich einstellenden Eigendruck des Reaktionsgemisches oder einem geringfügig höheren Druck, durchgeführt.

Die Ausgangsstoffe können im stöchiometrischen Molverhältnis oder im Überschuß einer Komponente zur anderen eingesetzt werden. Bevorzugt setzt man O,2 - 2 Mol Ausgangsstoff II je Mol Epoxid ein.

Die eingesetzte Menge an Katalysator sollte über O,OOO1 Mol je Mol Ausgangsstoff II liegen. Im Hinblick auf eine hinreichend große Reaktionsgeschwindigkeit und einen nicht zu hohen Katalysatorverbrauch setzt man O,OO1 - O,2 Mol, bevorzugt O,OO1 - O,O5 Mol Katalysator je Mol Ausgangsstoff II ein.

Die Verwendung eines Lösungsmittels ist nicht notwendig, aber möglich, soweit sich dieses unter den Reaktionsbedingungen inert verhält. Als inerte Lösungsmittel können aliphatische und aromatische Kohlenwasserstoffe und Halogenkohlenwasserstoffe, Alkylether, Carbonsäureester, Nitrile und niedere Ketone eingesetzt werden.

Die Erfindung wird durch die nachfolgenden Beispiele näher erläutert, ohne hierdurch eingeschränkt zu werden.

Beispiele 1 - 5

18,73 g 3-Chlor-Pentandion-2,4, 4,4 g Ethylenoxid und 75 mg $Ni(Acetylacetonat)_2$ wurden in einem Autoklaven unter den in Tab. 1 angegebenen Bedingungen erhitzt. Die Reaktionsausträge wurden gaschromatographisch untersucht und enthielten die in der Tabelle angegebenen Konzentrationen an 5-Acetoxy-3-chlor-pentanon-2.

Tabelle 1

Temperatur und Reaktionszeit

| Nr. | T/°C | t/h | Ausgangsstoff/% | Produkt/% |
|---|---|---|---|---|
| 1 | 80 | 48 | 18,9 | 59,3 |
| 2 | 90 | 36 | 20,2 | 55,0 |
| 3 | 100 | 24 | 21,9 | 48,4 |
| 4 | 100 | 8 | 41,7 | 30,7 |
| 5 | 120 | 8 | 27,2 | 44,6 |

Beispiel 6

In einem Autoklaven wurden 47,1 g 3-Chlorpentandion-2,4, 3O,8 g Ethylenoxid, 5O g Chlorbenzol (als Lösungsmittel), 1 g Nickel(II)acetylacetonat und 1 g Tetrabutylammoniumchlorid unter einem Druck von 5O bar Stickstoff 12 h lang auf 14O°C erhitzt. Nach Destillation des Austrags erhielt man 22,5 g Chlorpentandion (48 % des Einsatzes) und 2O,4 g 5-Acetoxy-3-chlor-pentanon-2 vom Kp. 6O°C/O,1 mbar (Ausb. 32 %, Selektivität 62 %).

Beispiele 7 - 1O

6,73 g 3-Chlorpentandion-2,4, 4,4 g Ethylenoxid, 12 g Lösungsmittel und 75 mg $Ni(acac)_2$ wurden in einem Autoklaven unter den in Tab. 2 angegebenen Bedingungen erhitzt. Die Reaktionsausträge wurden gaschromatographisch untersucht und enthielten die in der Tabelle angegebenen Konzentrationen an 5-Acetoxy-3-chlor-pentanon-2.

Tabelle 2

Lösungsmittel

| Nr. | T/°C | t/h | Lösungsmittel | Ausgangs-stoff/% | Produkt/% |
|---|---|---|---|---|---|
| 7 | 110 | 8 | Trichlorethan | 2,3 | 58,0 |
| 8 | 90 | 8 | Aceton | 6,8 | 54,4 |
| 9 | 130 | 8 | Acetonitil | 4,2 | 23,9 |
| 10 | 150 | 12 | Cyclohexan | 1,7 | 20,2 |

Beispiele 11 - 16

18,73 g 3-Chlorpentandion-2,4, 4,4 g Ethylenoxid und 75 mg des in Tab. 3 angegebenen Katalysators wurden in einem Autoklaven unter den angegebenen Bedingungen erhitzt. Die Reaktionsausträge wurden gaschromatographisch untersucht und enthielten die in der Tabelle angegebenen Konzentrationen an 5-Acetoxy-3-chlor-pentanon-2.

Tabelle 3

Katalysatoren

| Nr. | T/°C | t/h | Katalysator | Ausgangs-stoff/% | Produkt/% |
|---|---|---|---|---|---|
| 11 | 100 | 24 | $SnCl_2(acac)_2$ | 29,8 | 15,5 |
| 12 | 100 | 24 | $TiCl_2(acac)_2$ | 31,2 | 24,3 |
| 13 | 100 | 24 | $Mg(acac)_2$ | 16,1 | 38,9 |
| 14 | 130 | 12 | $Pd(acac)_2$ | 6,7 | 28,4 |
| 15 | 130 | 12 | $Zr(acac)_4$ | 9,0 | 15,5 |
| 16 | 130 | 12 | $VO(acac)_3$ | 6,8 | 16,5 |

Beispiel 17

In einem Autoklaven wurden 52,9 g α-Chloracetessigsäuremethylester, 30,8 g Ethylenoxid, 84 g Trichlorethan (als Lösungsmittel) und 575 mg Nickel(II)acetylacetonat unter einem Druck von 50 bar Stickstoff 8 h lang auf 120°C erhitzt. Nach Destillation des Austrags erhielt man 27,0 g Ausgangsstoff (51 % des Einsatzes) und 15,4 g 4-Acetoxy-2-chlor-butansäuremethylester vom Kp. 56°C/0,1 mbar (Ausb. 24 %, Selektivität 49 %).

Beispiele 18 - 22

7,5 g α-Chloracetessigsäure-methylester, 4,4 g Ethylenoxid, 12 g Lösungsmittel und 75 mg $Ni(acac)_2$ wurden in einem Autoklaven 8 Stunden unter den in Tab. 4 angegebenen Bedingungen erhitzt. Die Reaktionsausträge wurden gaschromatographisch untersucht und enthielten die in der Tabelle angegebenen Konzentrationen an 4-Acetoxy-2-chlor-butansäure-methylester.

Tabelle 4

Lösungsmittel

| Nr. | T/°C | Lösungsmittel | Ausgangs-stoff/% | Produkt/% |
|---|---|---|---|---|
| 18 | 110 | Trichlorethan | 9,6 | 40,1 |
| 19 | 110 | Tetrachlormethan | 7,9 | 37,7 |
| 20 | 130 | Tetrachlorethan | 6,1 | 37,1 |
| 21 | 130 | Trichlormethan | 16,7 | 23,8 |
| 22 | 110 | Dichlormethan | 8,4 | 40,1 |

**Patentansprüche**

1. Verfahren zur Herstellung von α-Chlor-carbonylverbindungen der allgemeinen Formel I

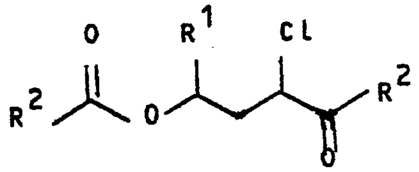

(I)

worin $R^1$ für Wasserstoff oder eine Alkylgruppe mit 1 bis 6 Kohlenstoffatomen steht und die Reste $R^2$ gleich oder verschieden sind und eine Alkylgruppe mit 1 bis 4 Kohlenstoffatomen oder Alkoxygruppe mit 1 bis 4 Kohlenstoffatomen darstellen, dadurch gekennzeichnet, daß man eine α-Chlor-ß-dicarbonylverbindung der allgemeinen Formel II

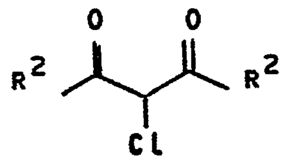

(II)

worin die Reste $R^2$ die vorstehenden Bedeutungen besitzen, mit einem Epoxyd der allgemeinen Formel III

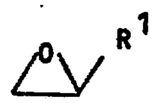

(III)

worin $R^1$ die vorstehenden Bedeutungen besitzt, bei einer Temperatur im Bereich von 2O bis 2OO°C in Gegenwart eines Metallacetylacetonats als Katalysator umsetzt.

2. Verfahren nach Anspruch 1, dadurch gekennzeichnet, daß man Acetylacetonate der Metalle Al, V, Mn, Mg, Sn, Co, Ni, Fe, Cu, Zr und Pd, einsetzt.

3. Verfahren nach einem der Ansprüche 1 oder 2, dadurch gekennzeichnet, daß man O,OO1 bis O,2 Mol Katalysator pro Mol α-Chlor-ß-dicarbonylverbindung der allgemeinen Formel II verwendet.

4. Verfahren nach Anspruch 3, dadurch gekennzeichnet, daß man O,OO1 bis O,O5 Mol Katalysator pro Mol α-Chlor-ß-dicarbonylverbindung der allgemeinen Formel II einsetzt.

5. Verfahren nach einem der Ansprüche 1 bis 4, dadurch gekennzeichnet, daß man O,2 bis 2 Mol α-Chlor-ß-dicarbonylverbindung der allgemeinen Formel II pro Mol Epoxid der allgemeinen Formel III einsetzt.

6. Verfahren nach einem der Ansprüche 1 bis 5, dadurch gekennzeichnet, daß man in Gegenwart eines Lösungsmittels, ausgewählt unter aliphatischen und aromatischen Kohlenwasserstoffen, aliphatischen und aromatischen Halogenkohlenwasserstoffen, Alkylethern, Carbonsäureestern, Nitrilen und niederen Ketonen, arbeitet.

## Claims

1. A process for preparing an α-chlorocarbonyl compound of the general formula I

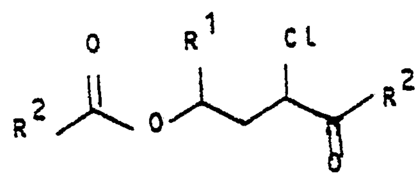

(I)

where $R^1$ is hydrogen or alkyl of 1 to 6 carbon atoms and each $R^2$ is identical to or different from the others and is alkyl of from 1 to 4 carbon atoms or alkoxy of from 1 to 4 carbon atoms, which comprises reacting an α-chloro-β-dicarbonyl compound of the general formula II

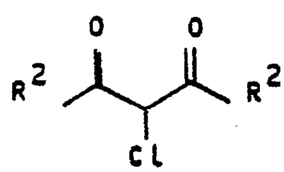

(II)

where each $R^2$ is as defined above, with an epoxide of the general formula III

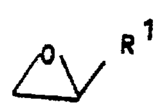

(III)

where $R^1$ is as defined above, at from 20 to 200°C in the presence of a metal acetylacetonate as catalyst.

2. A process as claimed in claim 1, wherein the acetylacetonate of the metal Al, V, Mn, Mg, Sn, Co, Ni, Fe, Cu, Zr or Pd is used.

3. A process as claimed in either of claims 1 and 2, wherein from 0.001 to 0.2 mole of catalyst is used per mole of α-chloro-β-dicarbonyl compound of the general formula II.

4. A process as claimed in claim 3, wherein from 0.001 to 0.05 mole of catalyst is used per mole of α-chloro-β-dicarbonyl compound of the general formula II.

5. A process as claimed in any of claims 1 to 4, wherein from 0.2 to 2 moles of α-chloro-β-dicarbonyl compound of the general formula II are used per mole of epoxide of the general formula III.

6. A process as claimed in any of claims 1 to 5, wherein the reaction is carried out in the presence of a solvent selected from the group consisting of aliphatic and aromatic hydrocarbons, aliphatic and aromatic halohydrocarbons, alkyl ethers, carboxylic esters, nitriles and lower ketones.

**Revendications**

1. Procédé de préparation de composés α-chlorocarbonylés de formule générale I

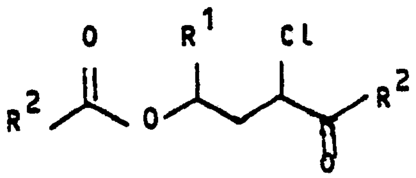

(I)

dans laquelle $R^1$ est mis pour un atome d'hydrogène ou un groupement alkyle de 1 à 6 atomes de carbone et les restes $R^2$ sont identiques ou différents et représentent des groupements alkyle de 1 à 4 atomes de carbone ou alcoxy de 1 à 4 atomes de carbone, caractérisé en ce qu'on fait réagir un composé α-chloro-β-dicarbonylé de formule générale II

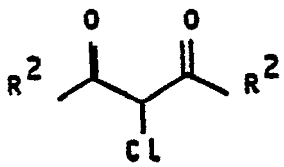

(II)

dans laquelle les restes $R^2$ ont les significations précédentes, avec un époxyde de formule générale III

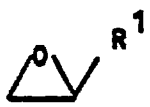

(III)

dans laquelle $R^1$ a la signification précédente, à une température de 20° à 200°C en présence d'un acétylacétonate de métal comme catalyseur.

2. Procédé selon la revendication 1, caractérisé en ce qu'on met en réaction des acétylacétonates des métaux Al, V, Mn, Mg, Sn, Co, Ni, Fe, Cu, Zr et Pd.

3. Procédé selon l'une ou l'autre des revendications 1 et 2, caractérisé en ce qu'on utilise de 0,001 à 0,2 mole de catalyseur par mole de composé α-chloro-β-dicarbonylé de formule générale II.

4. Procédé selon la revendication 3, caractérisé en ce qu'on met en réaction 0,001 à 0,05 mole de catalyseur par mole de composé α-chloro-β-dicarbonylé de formule générale II.

5. Procédé selon l'une des revendications 1 à 4, caractérisé en ce qu'on met en réaction 0,2 à 2 moles de composé α-chloro-β-dicarbonylé de formule générale II par mole d'époxyde de formule générale III.

6. Procédé selon l'une des revendications 1 à 5, caractérisé en ce qu'on travaille en présence d'un solvant choisi parmi les hydrocarbures aliphatiques et aromatiques, les hydrocarbures halogénés aliphatiques et aromatiques, les éthers alkyliques, les esters carboxyliques, les nitriles et les cétones inférieures.